(19) Europäisches Patentamt
European Patent Office
Office européen des brevets

(11) Veröffentlichungsnummer: **0 045 494**
**B1**

# (12) EUROPÄISCHE PATENTSCHRIFT

(45) Veröffentlichungstag der Patentschrift: **14.12.83**

(51) Int. Cl.³: **C 08 F 2/50**, A 61 K 6/08, C 08 F 20/12

(21) Anmeldenummer: **81106011.0**

(22) Anmeldetag: **30.07.81**

(54) **Photopolymerisierbare Masse, insbesondere für Dentalzwecke.**

(30) Priorität: **01.08.80 DE 3029276**

(43) Veröffentlichungstag der Anmeldung: **10.02.82 Patentblatt 82/6**

(45) Bekanntmachung des Hinweises auf die Patenterteilung: **14.12.83 Patentblatt 83/50**

(84) Benannte Vertragsstaaten: **AT BE CH DE FR GB IT LI NL SE**

(56) Entgegenhaltungen: **EP - A - 0 012 535**

(73) Patentinhaber: **Etablissement Dentaire IVOCLAR, FL-9494 Schaan (LI)**

(72) Erfinder: **Michl, Rudy, Dr., Troexlegass 17, FL-9494 Schaan (LI)**
Erfinder: **Willi, Hans Peter, Chem. HTL, Eggasweg 6, FL-9490 Vaduz (LI)**

(74) Vertreter: **Reitzner, Bruno, Dr. et al, Patentanwälte Dipl.-Ing. R. Splanemann Dr. B. Reitzner Tal 13, D-8000 München 2 (DE)**

**EP 0 045 494 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Photopolymerisierbare Masse, insbesondere für Dentalzwecke

Die Erfindung betrifft photopolymerisierbare Massen, insbesondere für Dentalzwecke, enthaltend photopolymerisierbare Olefinverbindungen, insbesondere Acryl- und/oder Methacrylverbindungen, sowie mindestens ein Keton und ein Amin als aktivierende Substanzen.

Es sind photopolymerisierbare Massen auf der Basis von Olefinverbindungen, insbesondere von Acryl- und/oder Methacrylverbindungen bekannt, die Ketone oder α,α-Diketone, die sichtbares Licht im Wellenlängenbereich von 300 bis 500 nm absorbieren, enthalten. Wichtige Vertreter dieser Ketone sind Benzophenon, Benzil und Campherchinon. Ein wichtiges Anwendungsgebiet für diese photopolymerisierbaren Massen ist das Dentalgebiet.

Bei der Herstellung von z.B. Lacken, Papierüberzügen oder Zahnfüllungen können die photopolymerisierbaren Massen allein mit diesen Ketonen katalysiert und durch Bestrahlung mit sichtbarem Licht ausgehärtet werden. Dabei werden relativ dünne Schichten ausgehärtet, wobei dies relativ lange dauert. Um die Härtungszeit zu verkürzen, ist es bekannt, in den photopolymerisierbaren Massen zusätzlich zu den Ketonen Amine mit α-ständigen CH-Gruppierungen zuzusetzen. Beispiele für derartige Amine sind Triäthanolamin, Dimethylaminoäthanol und Dimethylaminoäthylmethacrylat.

Diese Amine haben einen unangenehmen Geruch; ausserdem ist ihre Giftigkeit bzw. ihre relativ schwache Beschleuniger- oder Aktivatorwirkung nachteilig. Im allgemeinen bewirken sie ungefähr nur eine Verdoppelung der Reaktionsgeschwindigkeit.

In den photopolymerisierbaren Massen haben eine deutlich bessere Beschleunigerwirkung die N,N-Dimethylaniline, wie N,N-Dimethyl-p-toluidin oder N,N-Dimethyl-sym.-xylidin; der Zusatz dieser Substanzen führt jedoch manchmal zu starken Braunverfärbungen des erhaltenen Polymerisats, wobei auch hier die Beschleunigerwirkung noch nicht zufriedenstellend ist.

Der Erfindung liegt die Aufgabe zugrunde, photopolymerisierbare Massen der eingangs definierten Art zur Verfügung zu stellen, die sich leicht aushärten lässt, so dass z.B. auch verhältnismässig opake, mit Füllstoff beladene Massen in kurzer Zeit und in verhältnismässig dicken Schichten ausgehärtet werden können, ohne dass Geruchsbelästigungen entstehen.

Die Aufgabe wird erfindungsgemäss durch photopolymerisierbare Massen gelöst, die dadurch gekennzeichnet sind, dass sie ein Amin der allgemeinen Formel

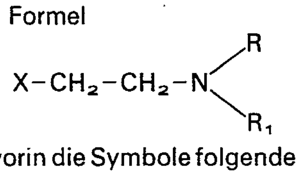

enthalten, worin die Symbole folgende Bedeutung haben:

X bedeutet Cyan, Chlor, Brom oder Jod,

R bedeutet die Gruppe $X-CH_2-CH_2-$ (worin X die vorstehend angegebene Bedeutung hat), eine Alkylgruppe mit 1 bis 6 C-Atomen oder eine Hydroxyalkylgruppe mit 2 bis 6 Kohlenstoffatomen, eine Acryl, Methacryl- oder Vinylgruppe,

$R_1$ bedeutet einen ggf. durch Chlor, Brom oder Jod bzw. durch Carboxy-, Carbalkoxy-, niedere Alkyl-, Alkoxy- oder Hydroxyalkyl-, Cyan- oder Nitrogruppen substituierten Phenyl- oder Benzyl- oder Styrylrest, oder

R und $R_1$ bilden zusammen eine Alkylengruppe mit 4 oder 5 C-Atomen,

wobei das Amin auch in polymerer Form vorliegen kann, wenn im Monomer R eine Acryl-, Methacryl- oder Vinylgruppe und/oder $R_1$ den Styrylrest darstellen.

Wenn das Amin in polymerer Form vorliegt, ist es im allgemeinen aus bis zu 1000 (vorzugsweise bis zu 200) wiederkehrenden Einheiten zusammengesetzt. Die photopolymerisierbaren Massen erfüllen insbesondere die Forderungen, die an Dentalkunststoffe gestellt werden. Die Polymerisationsgeschwindigkeit dieser Massen wird gegenüber der Verwendung der genannten Ketone allein etwa vervierfacht, was besonders wichtig ist, wenn Dentalkunststoffe, z.B. Zahnfüllungen, im Munde des Patienten durch Photopolymerisation ausgehärtet werden sollen. Die photopolymerisierbaren Dentalkunststoffmassen enthalten im allgemeinen Füllstoffe und Farbpigmente, wodurch die Intensität des zur Polymerisation verwendeten Lichtes stark geschwächt wird. Unter Dentalkunststoffmassen werden ausser Zahnfüllungsmaterialien und Zementen auch Massen zur Herstellung von künstlichen Zähnen, Kronen und Brücken sowie Prothesen verstanden, wobei diese Massen ausserhalb des Mundes durch Photopolymerisation ausgehärtet werden können.

Die photopolymerisierbaren Massen sind aber auch auf anderen medizinisch-technischen bzw. rein technischen Gebieten anwendbar, wo es auf eine schnelle Aushärtung ankommt. Beispiele für derartige Anwendungsgebiete finden sich in der Druck-, Lack-, Photographie- bzw. Kopierindustrie, wo Kunststoffschichten möglichst schnell ausgehärtet werden sollen.

Von den in den erfindungsgemässen Massen verwendeten Aminen werden diejenigen bevorzugt, in denen X Cyan oder Chlor bedeutet; ferner stellt R vorzugsweise eine niedere Alkylgruppe mit 1 bis 5 C-Atomen, insbesondere die Methylgruppe, dar. $R_1$ ist vorzugsweise ein Phenylrest.

Beispiele für geeignete 2-Cyanäthylaminderivate sind N-Methyl-N-cyanäthylanilin, N,N-Di-(2-cyanäthyl)anilin, N,N-Di(2-cyanäthyl)-m-toluidin u. dgl. In diesen Beispielen bedeutet $R_1$ einen einfachen bzw. nur durch eine Methylgruppe substituierten Phenylrest. Beispiele für Verbindungen, in denen R und $R_1$ zusammen eine Alkylengruppe mit 4 bzw. 5 C-Atomen bilden, sind N-(2-Cyanäthyl)pyrrolidin und N-(2-Cyanäthyl)-piperidin.

Ein Beispiel, in welchem der Phenylrest von $R_1$ durch einen Benzylrest ersetzt ist, ist das N,N-Di-(2-cyanäthyl)benzylamin. In dieser Verbindungsklasse ist der Substituent natürlich ebenfalls nicht auf die Cyanäthylgruppe beschränkt, sondern kann alle vorstehend genannten Gruppen einschliessen.

Beispiele für Amine vom 2-Cyanäthyltyp, in denen der Rest $R_1$ einen substituierten Phenylrest darstellt, sind N,N-Di-(2-cyanäthyl)halogenaniline, wie N,N-Di-(2-cyanäthyl)-4-chloranilin, N,N-Di-(2-cyanäthyl)-3,5-dichloranilin und N,N-Di-(2-cyanäthyl)-2,6-dichloranilin sowie die N-Alkyl-N-(2-cyanäthyl)halogenaniline, wie N-Methyl-N-(2-cyanäthyl)-4-chloranilin, N-Methyl-N-(2-cyanäthyl)-3,5-dichloranilin und N-Methyl-N-(2-cyanäthyl)-2,6-dichloranilin.

In dieser Verbindungsgruppe kann der Phenylrest auch durch Alkylgruppen substituiert sein. Beispiele für derartige Verbindungen sind N,N-Di-(2-cyanäthyl)alkylaniline, wie N,N-Di-(2-cyanäthyl)-4-methylanilin, N,N-Di-(2-cyanäthyl)-3,5-dimethylanilin und N,N-Di-(2-cyanäthyl)-2,6-dimethylanilin sowie N,Alkyl-N-(2-cyanäthyl)alkylaniline, wie N-Methyl-N-(2-cyanäthyl)-4-methylanilin, N-Methyl-N-(2-cyanäthyl)-3,5-dimethylanilin und N-Methyl-N(2-cyanäthyl)-2,6-dimethylanilin.

Die Alkylsubstituenten können hierbei verzweigt oder geradkettig sein. Weitere Substituenten am Phenylrest $R_1$ sind Brom, Jod, Carboxy-, Carbalkoxy-, niedere Alkoxy- oder Hydroxyalkyl-, Cyan- oder Nitrogruppen. Diese können an jeder beliebigen Substitutionsstelle stehen.

Beispiele für geeignete 2-Halogen-äthylaminderivate sind N-(2-Chloräthyl)-n-methylanilin, N-(2-Bromäthyl)-n-methylanilin, N,N-Di-(2-chloräthyl)anilin, N,N-Di-(2-bromäthyl)anilin, N,N-Di-(2-chloräthyl)-m-toluidin und N,N-Di-(2-bromäthyl)-m-toluidin. In diesen Beispielen bedeutet $R_1$ einen einfachen bzw. einen durch eine Methylgruppe substituierten Phenylrest.

Als Beispiele für Amine, in denen R und $R_1$ zusammen eine Alkylengruppe mit 5 C-Atomen bilden, seien N-(2-Chloräthyl)piperidin und N-(2-Bromäthyl)piperidin genannt.

Als Beispiele, in denen $R_1$ einen Benzylrest darstellt, seien N,N-Di-(2-chloräthyl)benzylamin und N,N-Di-(2-bromäthyl)benzylamin genannt.

Weitere Beispiele für 2-Halogenäthylaminderivate, in denen $R_1$ einen substituierten Phenylrest darstellt, sind, N,N-Di-(2-halogenäthyl)alkylaniline, wie N,N-Di-(2-chloräthyl)-4-methylanilin, N,N-Di-(2-chloräthyl)-3,5-dimethylanilin und N,N-Di-(2-chloräthyl)-2,6-dimethylanilin sowie N-Alkyl-N-(2-chloräthyl)alkylaniline, wie N-Methyl-N-(2-chloräthyl)-4-methylanilin, N-Methyl-N-(2-chloräthyl)-3,5-dimethylanilin und N-Methyl-N-(2-chloräthyl)-2,6-dimethylanilin.

Auch bei den 2-Halogenäthylaminderivaten können als Alkylsubstituenten sowohl am Stickstoffatom wie am Phenylrest auch höhere verzweigte oder geradkettige Alkylreste verwendet werden, desgleichen auch die entsprechenden Hydroxyalkylreste. Weiterhin sind am Phenyl- bzw. Benzylrest als Substituenten Jod, Carboxy-, Carbalkoxy-, Alkoxy-, Cyan- oder Nitrogruppen an jeder beliebigen Substitutionsstelle möglich.

Als Monomere können die üblichen photopolymerisierbaren Olefinverbindungen, insbesondere die Acryl- und/oder Methacrylverbindungen, verwendet werden, vorzugsweise folgende Verbindungen:

Äthylenglykoldimethacrylat
Butandiol-1,4-dimethacrylat
Triäthylenglykoldimethacrylat
Dodekandiol-1,12-dimethacrylat
Dekandiol-1,10-dimethacrylat
2,2-Bis[-p(γ-methacryloxy-β-hydroxypropoxy)-phenyl]propan
Diaddukt aus 2-Hydroxyäthylmethacrylat und Trimethylhexamethylendiisocyanat
Diaddukt aus 2-Hydroxyäthylmethacrylat und Isophorondiisocyanat
Trimethylolpropantrimethacrylat
Pentaerythrittrimethacrylat
Pentaerythrittetramethacrylat

Von den genannten Verbindungen können auch die entsprechenden Acrylate anstelle der Methacrylate eingesetzt werden.

Beispiele für andere geeignete Olefinverbindungen, die in den polymerisierbaren Massen eingesetzt werden können, sind ungesättigte Polyester, d.h. Mischkondensate aus ungesättigten Carbonsäuren und mehrwertigen Alkoholen, ggf. in Kombination mit Methacrylaten.

Den photopolymerisierbaren Massen können auch Füllstoffe zugesetzt werden. Für Dentalzwecke kommen hierbei insbesondere inerte, anorganische Füllstoffe in Betracht, wie z.B. die Oxide von Aluminium und Silicium, Silicatgläser, Calciumcarbonat und anderes mehr. Ausgezeichnete Füllstoffe sind die pyrogenen Kieselsäuren, deren BET-Oberfläche zwischen 30 und 400 $m^2/g$ liegen kann. Grundsätzlich ist es aber auch möglich, Füllstoffe einzusetzen, die durch Fällung erzeugt wurden. Während die mittlere Korngrösse der Primärteilchen bei den pyrogen erzeugten Füllstoffen eine Obergrenze von 70 nm nicht übersteigt, sind für die Fällungsfüllstoffe Teilchengrössen bis 10 μm, insbesondere bis 1 μm möglich.

In den erfindungsgemässen photopolymerisierbaren Massen sind die aktivierenden Substanzen, nämlich die Ketone und Amine, im allgemeinen in Mengen von etwa 0,05 bis 70 Gew.-%, bezogen auf die gesamte Masse, enthalten. Die angegebene Obergrenze kann dann erreicht werden, wenn man ein polymerisierbares Amin auswählt, d.h. ein solches, in dem R eine Acryl-, Methacryl- oder Vinylgruppe und/oder $R_1$ eine Styrylgruppe darstellt. Ein solches Amin kann zunächst für sich polymerisiert und anschliessend der photopolymerisierbaren Masse zugesetzt werden. Es kann aber auch in seiner monomeren Form zusammen mit den photopolymerisierbaren Olefinverbindungen photopolymerisiert werden, wobei in beiden Fällen das Keton bzw. Diketon als Katalysator dient. Selbst in der polymeren Form haben diese Amine noch eine Beschleunigerwirkung. Vorzugsweise werden die aktivierenden Substanzen

in Mengen von etwa 0,05 bis 5 Gew.-% eingesetzt. Spezielle Bereiche liegen zwischen 0,1 und 3 insbesondere zwischen 0,2 und 0,8 Gew.-%. Das Keton, bzw. das α,α-Diketon liegt üblicherweise in Mengen von etwa 5 bis 45, vorzugsweise in Mengen von etwa 10 bis 35 Gew.-%, bezogen auf die gesamte aktivierende Substanz vor. Als Ketone, bzw. α,α-Diketone können substituierte Benzophenone und Benzile verwendet werden, vorzugsweise Verbindungen sind aber Benzophenon, Benzil und/oder Campherchinon.

Die photopolymerisierbaren Massen enthalten als aktivierende Substanzen vorteilhaft ein Gemisch aus N-Methyl-N-Cyanäthylanilin und D,L-Campherchinon.

Die photopolymerisierbaren Massen werden vorzugsweise wie folgt hergestellt und ausgehärtet: Man stellt eine Paste her, indem man in einem Mischgerät folgende Substanzen zusammenmischt:

Ein oder mehrere polymerisierbare Methacrylate, einen pulverförmigen Füllstoff, der entweder organisch oder anorganisch sein kann, eine aktivierende Substanz, z.B. ein Gemisch aus N-Methyl-N-Cyanäthylanilin und D,L-Campherchinon, ggf. Farbpigmente. Diese Paste ist lichtempfindlich und wird z.B. vom Zahnarzt als ein Füllungsmaterial verwendet. Man lagert diese Paste in lichtundurchlässigen Behältern. Nachdem der Zahnarzt eine Kavität präpariert hat, wird die Paste in diese eingebracht und in die gewünschte Form modelliert. Anschliessend wird die Paste mit einer Halogenkaltlichtleuchte etwa 20 bis 60 s bestrahlt und so polymerisiert.

Dieselbe Paste oder auch andere mit anderen Zusammensetzungen können zum Modellieren von Kronen bzw. Brücken verwendet werden. Die Polymerisation erfolgt dann in einem Druckpolymerisationsgerät, welches z.B. mit Wasser gefüllt wird und so beschaffen sein muss, dass die polymerisierbare Masse mit Hilfe von Licht ausgehärtet werden kann. Zu diesem Zweck besteht das Gerät z.B. zumindest teilweise aus Glas.

Die Erfindung ist durch die nachstehenden Beispiele in nicht einschränkender Weise erläutert. Als Campherchinon wurde jeweils der D,L-Typ eingesetzt.

*Beispiel 1*

Zur Untersuchung der Reaktionsgeschwindigkeiten verschiedener photopolymerisierbarer Masssen wurden die Aktivatoren in einem Gemisch aus 80 Gew.-% Diaddukt aus 2-Hydroxyäthylmethacrylat und Trimethylhexamethylendiisocyanat und 20 Gew.-% Triäthylenglykoldimethacrylat gelöst.

Im einzelnen wurden D,L-Campherchinon und Triäthanolamin, Campherchinon und N-Methyl-N-2-cyanäthylanilin und Campherchinon und N,N-Dimethyl-p-toluidin in die photopolymerisierbaren Massen eingearbeitet, wobei jeweils eine Konzentration von 0,01 mol/kg eingehalten wurde. Dies entspricht z.B. 1,66 Campherchinon/kg und 3,2 g N-Methyl-N-cyanäthylanilin/kg Lösung. Die photopolymerisierbaren Massen wurden in kleine Kunststofftöpfchen (8 mm hoch, 16 mm im Durchmesser) im Abstand von 10 cm mit einer 20-W-Halogenlampe (Osram 41900) bestrahlt. Mittels einer Stoppuhr und einem feinen Spatel wurde die jeweilige Polymerisationszeit bestimmt wobei das Ende der Polymerisationszeit wie folgt festgelegt wurde:

In Abständen von 4 s wurde der Spatel in die Probe geführt. Das Ende der Polymerisation ist dabei an der vollständigen harten Oberfläche der Probe zu erkennen.

Die Polymerisationszeit beträgt:

1. für Campherchinon 96 s
2. für Campherchinon/Triäthanolamin 48 s
3. für Campherchinon/N,N-Dimethyl-p-toluidin 30 s
4. für Campherchinon/N-Methyl-N-(2-cyanäthyl)anilin 24 s

Die Probe 4 war ausserdem praktisch geruchsfrei, während die Proben 2 und 3 unangenehm rochen.

*Beispiel 2*

Das im Beispiel 1 verwendete Methacrylatgemisch mit 0,01 mol Campherchinon/kg und 0,01 mol Aktivator/kg wurde mit 75 Gew.-% feingemahlenem Quarzpulver, wie es üblicherweise für Zahnfüllungsmaterialien verwendet wird, gemischt. Zusätzlich wird noch 0,2% Titandioxidpaste Irgalith Dispers Weiss 1123, Ciba Geigy zugemischt, um eine Transparenz von 28% zu erreichen.

Diese lichtempfindliche Paste wird nun in eine Prüfform aus Polyoxymethylen eingefüllt, die wie folgt beschaffen ist:

Zwei Klötzchen von 40×30×15 mm, wovon eine auf der grössten Fläche mit einer durchgehenden Rinne von 3,5×3,5 mm versehen ist. Die beiden Klötzchen werden nun mit Hilfe einer Schraubzwinge so zusammengehalten, dass ein Klotz von 40×30×30 mm entsteht, in dessen Mitte sich eine Kavität von 3,5×3,5 mm befindet.

Nach dem Füllen der Prüfform wird die Paste in Richtung der Achse der Kavität 20 s mit einer Faserleiterhalogenlampe (Pluraflex HL 150 Litema München) mit Blaulicht bestrahlt. Danach wird die Prüfform geöffnet, und die Dicke der gehärteten Schicht wird mit einer Schieblehre gemessen.

Sie beträgt für:

| | | |
|---|---|---|
| Paste Nr. 1) | Campherchinon: | 0,9-1,1 mm |
| Paste Nr. 2) | Campherchinon Triäthanolamin: | 1,9-2,1 mm |
| Paste Nr. 3) | Campherchinon N,N-Dimethyl-3,5 Xylidin: | 4,2-4,5 mm |
| Paste Nr. 4) | Campherchinon N(2-Cyanäthyl)N-methylanilin: | 4,8-5,2 mm |

Die Pasten Nrn. 1 bis 3 sind weniger durchgehärtet als Paste 4, was auf die erhöhte Aktivität des darin verwendeten Amins hinweist. Ausserdem sind die Pasten Nrn. 2 und 3 sehr unangenehm geruchsintensiv, während die Pasten 1 und 4 praktisch geruchlos sind.

Mit den vorstehend genannten Pasten wurden durch Bestrahlen mit Halogenlicht Prüfkörper (kreisrund Plättchen von 1 mm Dicke und 10 mm Durchmesser) hergestellt. Diese Plättchen wurden 3 Wochen bei 60°C in destilliertem Wasser gelagert. Der aus Paste Nr. 3 hergestellte Prüfkörper hatte sich stark braun verfärbt, während der aus Paste Nr. 2 hergestellte Prüfkörper nur wenig verfärbt war. Die aus Paste Nrn. 1 und 4 hergestellten Prüfkörper waren nicht verfärbt.

*Beispiel 3*

Es wurden andere Aktivatorenkombinationen verwendet, ansonsten wurde nach Beispiel 2 gearbeitet. Die Ergebnisse sind in Tabelle I angegeben.

Tabelle I

| Initiator | Aktivator | Geruch | Polymerisationstiefe in mm | Verfärbung 3 Wochen 60°C dest. Wasser |
|---|---|---|---|---|
| Campherchinon | N,N-Dimethylbenzylamin | unangenehm | 4,8-5,1 | braungelb |
| Campherchinon | N,N-Di-(2-cyanäthyl)anilin | geruchlos | 4,6-4,8 | keine Verfärbung |
| Campherchinon | N,N-Dimethylaminoadamantan | leichter Amingeruch | 3,1-3,3 | keine Verfärbung |

*Beispiel 4*

Man stellt ein polymerisierbares Gemisch aus folgenden Komponenten her:

37,5 Gew.-% 2,2 Bis[p-(γ-methacryloxy-β-hydroxypropoxy)phenyl]propan

25 Gew.-% Diaddukt aus 2-Hydroxyäthylmethacrylat und Trimethylhexamethylendiisocyanat

37,5 Gew.-% Triäthylenglykoldimethacrylat

Zu dieser polymerisierbaren Mischung gibt man 0,25% Campherchinon und 0,75% eines Amins hinzu, wie es aus der nachstehenden Tabelle hervorgeht. Man mischt 60 Gew.-% dieser Mischung mit 40 Gew.-% einer pyrogenen Kieselsäure (Aerosil OX 50 der Firma Degussa), die eine BET-Oberfläche von 50 $m^2/g$ aufweist. Durch intensives Rühren erhält man eine zähflüssige Masse. Man nimmt einen Teil dieser zähflüssigen Masse, setzt 1% Benzoylperoxid zu und polymerisiert das Ganze bei 100°C während 12 h. Durch anschliessendes intensives Zerkleinern enthält man einen Polymerfüller, der selbst mit dem anorganischen Füllstoff angereichert ist.

Man mischt diesen Polymerfüller mit der am Anfang hergestellten zähflüssigen Masse im Gewichtsverhältnis 1:1 in einem geeigneten Kneter und erhält eine homogene Paste. Um eine Transparenz von 28% zu erreichen, wurde während des Knetens noch 0,2% einer Titandioxidpaste zugemischt. Mit dieser Paste wurden die Durchhärtungstiefen und die Verfärbungen wie in Beispiel 2 geprüft.

Die Ergebnisse sind in Tabelle II angegeben.

Tabelle II

| Initiator | Aktivator | Polymerisationstiefe in mm | Verfärbung 3 Wochen 60°C dest. $H_2O$ | Geruch |
|---|---|---|---|---|
| Campherchinon | — | 1,2-1,4 | keine | — |
| Campherchinon | Triäthanolamin | 2,0-2,2 | unbedeutend | unangenehm |
| Campherchinon | N,N-Dimethyl-3,5-xylidin | 4,5-4,8 | braungelb | unangenehm |
| Campherchinon | N-(2-Cyanäthyl)-N-methylanilin | 4,8-5,4 | keine | geruchlos |

**Patentansprüche**

1. Photopolymerisierbare Masse, insbesondere für Dentalzwecke, enthaltend photopolymerisierbare Olefinverbindungen, insbesondere Acryl- und/oder Methacrylverbindungen, sowie mindestens ein Keton und ein Amin als aktivierende Substanzen, dadurch gekennzeichnet, dass das Amin die allgemeine Formel

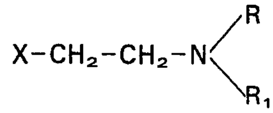

hat, worin die Symbole folgende Bedeutung haben:

X bedeutet Cyan, Chlor, Brom oder Jod,

R bedeutet die Gruppe $X-CH_2-CH_2-$ (worin X die vorstehend angegebene Bedeutung hat), eine Alkylgruppe mit 1 bis 6 C-Atomen oder eine Hydroxyalkylgruppe mit 2 bis 6 Kohlenstoffatomen, eine Acryl, Methacryl- oder Vinylgruppe,

$R_1$ bedeutet einen ggf. durch Chlor, Brom oder Jod bzw. durch Carboxy-, Carbalkoxy-, niedere Alkyl-, Alkoxy- oder Hydroxyalkyl-, Cyan- oder Nitrogruppen substituierten Phenyl-, Benzyl- oder Styrylrest, oder

R und $R_1$ bilden zusammen eine Alkylengruppe mit 4 oder 5 C-Atomen,
wobei das Amin auch in polymerer Form vorliegen kann, wenn im Monomer R eine Acryl-, Methacryl- oder Vinylgruppe und/oder $R_1$ den Styrylrest darstellen.

2. Photopolymerisierbare Masse nach Anspruch 1, dadurch gekennzeichnet, dass R eine niedere Alkylgruppe mit 1 bis 5 C-Atomen, insbesondere die Methylgruppe, und $R_1$ den Phenylrest darstellt.

3. Photopolymerisierbare Masse nach einem der beiden Ansprüche 1 oder 2, dadurch gekennzeichnet, dass sie ein oder mehrere, das Licht im Wellenlängenbereich von 300 bis 500 nm absorbierende Ketone oder α,α-Diketone enthält.

4. Photopolymerisierbare Masse nach einem der-Ansprüche 1 bis 3, dadurch gekennzeichnet, dass die aktivierenden Substanzen in Mengen von etwa 0,05 bis 70 Gew.-%, vorzugsweise in Mengen von 0,05 bis 5 Gew.-%, bezogen auf die gesamte Masse, vorliegen.

5. Photopolymerisierbare Masse, nach einem der Ansprüche 1 bis 4, dadurch gekennzeichnet, dass das Keton bzw. das α,α-Diketon in Mengen von etwa 5 bis 45 Gew.-%, bezogen auf die aktivierenden Substanzen, vorliegt.

6. Photopolymerisierbare Masse nach einem der Ansprüche 1 bis 5, dadurch gekennzeichnet, dass das Keton bzw. α,α-Diketon Benzophenon, Benzyl und/oder Campherchinon darstellt.

7. Photopolymerisierbare Masse nach einem der Ansprüche 1 bis 6, dadurch gekennzeichnet, dass sie als Keton D,L-Campherchinon und als Amin N-Methyl-N-Cyanäthylanilin enthält.

8. Photopolymerisierbare Masse nach einem der Ansprüche 1 bis 7, dadurch gekennzeichnet, dass sie mindestens einen Füllstoff enthält.

## Revendications

1. Matériau photopolymérisable, en particulier pour applications dentaires, renfermant des composés oléfiniques, notamment des composés acryliques et/ou méthacryliques, ainsi qu'au moins une cétone et une amine comme substances activantes, caractérisé en ce que l'amine répond à la formule générale:

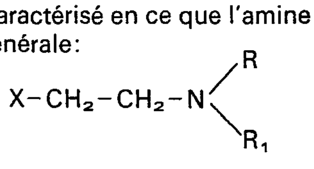

dans laquelle les symboles ont la signification suivante:

X représente le cyanogène, le chlore, le brome ou l'iode,

R représente le groupe $X-CH_2-CH_2-$ (où X a la signification indiquée précédemment), un groupe alcoyle comportant 1 à 6 atomes de carbone ou un groupe hydroxyalcoyle comportant 2 à 6 atomes de carbone, un groupe acryl, méthacryl ou vinyl,

$R_1$ représente un reste phényle, benzyle ou styryle, éventuellement substitué par du chlore, du brome ou de l'iode, ou par des groupes carboxy, carbalcoxy, alcoyle inférieur alcoxy ou hydroxyalcoyle, cyano ou nitro, ou

R et $R_1$ forment ensemble un groupe alcoylène comportant 4 ou 5 atomes de carbone,
l'amine pouvant également se présenter sous forme polymère lorsque, dans le monomère, R représente un groupe acryle, méthacryle ou vinyle et/ou $R_1$ représente le reste styryle.

2. Matériau selon la revendication 1, caractérisé en ce que R représente un groupe alcoyle inférieur comportant 1 à 5 atomes de carbone, notamment le groupe méthyle, et $R_1$ le reste phényle.

3. Matériau photopolymérisable selon l'une des revendications 1 ou 2, caractérisé en ce qu'il renferme une ou plusieurs cétones ou α,α-dicétones absorbant la lumière dans l'intervalle de longueurs d'onde de 300 à 500 nm.

4. Matériau selon l'une quelconque des revendications 1 à 3, caractérisé en ce que les substances activantes sont présentes dans des proportions d'environ 0,05 à 70% en poids, de préférence dans des proportions de 0,05 à 5% en poids, rapporté à la masse totale.

5. Matériau selon l'une quelconque des revendications 1 à 4, caractérisé en ce que la cétone ou l'α,α-dicétone est présente dans des proportions de 5 à 45% en poids, rapporté aux substances activantes.

6. Matériau selon l'une quelconque des revendications 1 à 5, caractérisé en ce que l'α-α-dicétone est représentée par la benzophénone, le benzyle et/ou la camphoquinone.

7. Matériau selon l'une quelconque des revendications 1 à 6, caractérisé en ce qu'il renferme comme cétone la D,L-camphoquinone et, comme amine, la N-méthyl-N-cyanéthylaniline.

8. Matériau selon l'une quelconque des revendications 1 à 7, caractérisé en ce qu'il renferme au moins une matière de charge.

## Claims

1. Photopolymerisable composition, in particular for dental purposes, containing photopolymerisable olefin compounds, in particular acrylic and/or methacrylic compounds, and also at least one ketone and one amine as activating substances, characterized in that the amine has the general formula:

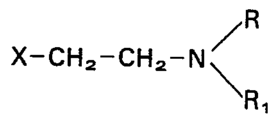

wherein the symbols have the following meaning:

X signifies cyano, chlorine, bromine or iodine,

R signifies the group $X-CH_2-CH_2-$ (wherein X has the meaning indicated above), an alkyl group with 1 to 6 carbon atoms or a hydroxyalkyl group with 2 to 6 carbon atoms, an acryl, methacryl or vinyl group,

$R_1$ signifies a phenyl, benzyl or styryl radical optionally substituted by chlorine, bromine or iodine, or by carboxy, carbalkoxy, lower alkyl, alkoxy or hydroxyalkyl, cyano or nitro groups, or

R and $R_1$ together form an alkylene group with 4 or 5 carbon atoms,

in which case the amine can also be present in polymeric form, if in the monomer R represents an acryl, methacryl or vinyl group and/or $R_1$ represents the styryl radical.

2. Photopolymerisable composition according to claim 1, characterized in that R represents a lower alkyl group with 1 to 5 carbon atoms, in particular the methyl group, and $R_1$ represents the phenyl radical.

3. Photopolymerisable composition according to one of claims 1 or 2, characterized in that it contains one or more ketones or α,α-diketones absorbing light in the wavelength range of from 300 to 500 nm.

4. Photopolymerisable composition according to one of claims 1 to 3, characterized in that the activating substances are present in quantities of from about 0.05 to 70% by weight, preferably in quantities of from 0.05 to 5% by weight, in relation to the total composition.

5. Photopolymerisable composition according to one of claims 1 to 4, characterized in that the ketone or the α,α-diketone is present in quantities of from about 5 to 45% by weight, in relation to the activating substances.

6. Photopolymerisable composition according to one of claims 1 to 5, characterized in that the ketone or α,α-diketone represents benzophenone, benzyl and/or camphorquinone.

7. Photopolymerisable composition according to one of claims 1 to 6, characterized in that as ketone it contains D,L-camphorquinone and as amine N-methyl-N-cyanoethyl aniline.

8. Photopolymerisable composition according to one of claims 1 to 7, characterized in that it contains at least one filler.